# EUROPEAN PATENT SPECIFICATION

(11) **EP 3 377 466 B1**
(45) Date of publication and mention of the grant of the patent: **30.06.2021**
(21) Application number: 16797584.6
(22) Date of filing: 17.11.2016
(51) Int. Cl.: C07C 29/132, C07C 31/20, C07C 29/60, B01J 23/40, B01J 23/46, B01J 25/02, B01J 23/68, B01J 23/70, B01J 21/08, B01J 35/00

(54) **CATALYST SYSTEM AND PROCESS FOR THE PRODUCTION OF GLYCOLS**
KATALYSATORSYSTEM UND VERFAHREN ZUR HERSTELLUNG VON GLYCOLEN
SYSTÈME DE CATALYSEUR ET PROCÉDÉ POUR LA PRODUCTION DE GLYCOLS

(30) Priority: 19.11.2015 EP 15195496
(43) Date of publication of application: 26.09.2018
(73) Proprietor: Shell Internationale Research Maatschappij B.V., 2596 HR The Hague (NL)
(72) Inventor: EDULJI, Smita, Houston, Texas 77082 (US)
(74) Representative: Shell Legal Services IP
(86) International application number: PCT/EP2016/078067
(87) International publication number: WO 2017/085219

(56) References cited:
- WO-A1-2015/028398
- WO-A2-2013/015955
- US-A1- 2011 046 419
- US-A1- 2012 172 633
- JI N ET AL: "Catalytic conversion of cellulose into ethylene glycol over supported carbide catalysts", CATALYSIS TODAY, ELSEVIER, NL, vol. 147, no. 2, 30 September 2009 (2009-09-30), pages 77-85, XP026470036, ISSN: 0920-5861, DOI: 10.1016/J.CATTOD.2009.03.012 [retrieved on 2009-04-17] cited in the application
- YUE LIU ET AL: "Tungsten trioxide promoted selective conversion of cellulose into propylene glycol and ethylene glycol on a ruthenium catalyst", ANGEWANDTE CHEMIE INTERNATIONAL EDITION, vol. 51, no. 13, 24 February 2012 (2012-02-24), pages 3249-3253, XP055103283, ISSN: 1433-7851, DOI: 10.1002/anie.201200351 cited in the application
- YU XIANG-RONG: "Rapid microdetermination of carbon and hydrogen in organic compounds - use of silver tungstate (Ag2WO4)-magnesium oxide/cobalt oxide (Co3O4)/silver tungstate (Ag2WO4)-magnesium oxide as the combustion tube packing", FENXI HUAXUE / CHINESE JOURNAL OF ANALYTICAL CHEMISTRY, vol. 9, no. 2, 1 January 1981 (1981-01-01) , pages 131-136, XP055676606, CN ISSN: 0253-3820

## Description

### Field of the Invention

The present invention relates to a process for the production of glycols, in particular monoethylene glycol and monopropylene glycol from a saccharide-containing feedstock and to a catalyst system for use in said process.

### Background of the Invention

Monoethylene glycol (MEG) and monopropylene glycol (MPG) are valuable materials with a multitude of commercial applications, e.g. as heat transfer media, antifreeze, and precursors to polymers such as polyethylene terephthalate (PET).

Said glycols are currently made on an industrial scale by hydrolysis of the corresponding alkylene oxides, which are the oxidation products of ethylene and propylene, generally produced from fossil fuels.

In recent years increased efforts have been focussed on reducing the reliance on fossil fuels as a primary resource for the provision of fuels and commodity chemicals. Carbohydrates and related 'biomass' are seen as key renewable resources in the efforts to provide new fuels and alternative routes to desirable chemicals.

In particular, certain carbohydrates can be reacted with hydrogen in the presence of a catalyst system to generate polyols and sugar alcohols. Current methods for the conversion of saccharides to glycols revolve around a hydrogenation/hydrogenolysis process.

Reported processes generally require a first catalytic species to perform the hydrogenolysis reaction, which is postulated to have a retro-aldol mechanism, and a second catalytic species for hydrogenation.

Processes for the conversion of cellulose to products including MEG are described in Angew. Chem. Int. Ed. 2008, 47, 8510-8513 and Catalysis Today 147 (2009), 77-85 using nickel-promoted tungsten carbide catalysts.

US 2011/0312487 A1 describes a process for generating at least one polyol from a saccharide-containing feedstock and a catalyst system for use therein, wherein said catalyst system comprises a) an unsupported component comprising a compound selected from the group consisting of a tungsten compound, a molybdenum compound and any combination thereof; and b) a supported compound comprising an active metal component selected from the group consisting of Pt, Pd, Ru, Rh, Ni, Ir, and combinations thereof on a solid catalyst support.

Examples of the unsupported catalyst component in US 2011/0312487 A1 are said to include tungstic acid (H₂WO₄), ammonium tungstate ((NH₄)₁₀H₂(W₂O₇)₆), ammonium metatungstate ((NH₄)₆H₂(W₁₂O₄₀)·xH₂O), ammonium paratungstate ((NH₄)₁₀[H₂W₁₂O₄₂]_{·}4H₂O), and tungstate, metatungstate and paratungstate compounds comprising at least Group I or II element.

Catalyst systems tested in US 2011/0312487 A1 utilise tungstic acid, tungsten oxide (WO₂), phosphotungstic acid (H₃PW₁₂O₄₀) and ammonium metatungstate as the unsupported catalyst component in conjunction with various nickel, platinum and palladium supported catalyst components.

US 2011/03046419 A1 describes a method for producing ethylene glycol from a polyhydroxy compound such as starch, hemicellulose, glucose, sucrose, fructose and fructan in the presence of catalyst comprising a first active ingredient and a second active ingredient, the first active ingredient comprising a transition metal selected from iron, cobalt, nickel, ruthenium, rhodium, palladium, iridium, and platinum, or a mixture thereof; the second active ingredient comprising a metallic state of molybdenum and/or tungsten, or a carbide, nitride, or phosphide thereof.

Angew. Chem. Int. Ed. 2012, 51, 3249-3253 describes a process for the selective conversion of cellulose into ethylene glycol and propylene glycol in the presence of a ruthenium catalyst and tungsten trioxide (WO₃).

AIChE Journal, 2014, 60 (11), pp. 3804-3813 describes the retro-aldol condensation of glucose using ammonium metatungstate as catalyst.

Continuous processes for generating at least one polyol from a saccharide-containing feedstock are described in WO 2013/015955 A, CN 103731258 A and WO 2015/028398 A1.

The products of the afore-mentioned processes are typically a mixture of materials comprising MEG, MPG, 1,2-butanediol (1,2-BDO) and other by-products.

One problem of catalyst systems in this field has been sensitivities to sulphur, chlorine and other contaminants typically present in the saccharide-containing feedstocks. Over time, this sensitivity can lead to reduced product yields.

It is highly desirable to develop catalyst systems for use in the conversion of saccharide-containing feedstocks which display reduced sensitivity to feedstock contaminants, and in particular sulphur, and which not only give improved overall yields of the desirable MEG and MPG products, but also have increased selectivity to MEG and fewer, or more desirable, by-products being produced.

### Summary of the Invention

The present invention has surprisingly found that certain catalyst systems display advantageous performance in the conversion of saccharide-containing feedstocks to polyols.

Accordingly, in a first aspect of the present invention there is provided a catalyst system comprising:
a) one or more catalytic species comprising silver and tungsten therein,
   wherein the one or more catalytic species comprising silver and tungsten therein are selected from silver tungstate-containing species and/or silver phosphotungstate -containing species; and
b) one or more catalytic species suitable for hydrogenation, wherein the one or more catalytic species suitable for hydrogenation are selected from one or more transition metals from Groups 8, 9 or 10 of the Periodic Table, and compounds thereof;
wherein the weight ratio of the one or more catalytic species comprising silver and tungsten therein to the one or more catalytic species suitable for hydrogenation is in the range of from 0.02:1 to 3000:1, on the basis of the total weight of the catalyst system.

In a further aspect of the present invention, there is provided a process for the preparation of monoethylene glycol from starting material comprising one or more saccharides, by contacting said starting material with hydrogen in a reactor in the presence of a solvent and said catalyst system.

### Brief Description of the Drawings

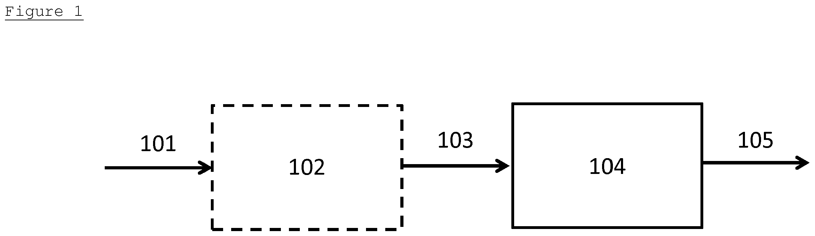

Figure 1 is a schematic diagram of an exemplary embodiment of the process of the invention.

### Detailed Description of the Invention

In the present invention, there has been surprisingly found a catalyst system which not only gives rise to advantageous yields of ethylene glycol and propylene glycol from saccharide-containing feedstocks, but which also shows improved tolerance to the presence of typical feedstock contaminants such as sulphur which ordinarily can result in catalyst poisoning.

In the catalyst system of the present invention, are present one or more catalytic species comprising silver and tungsten therein, wherein the one or more catalytic species comprising silver and tungsten therein are selected from silver tungstate-containing species and/or silver phosphotungstate -containing species; and one or more catalytic species suitable for hydrogenation, wherein the one or more catalytic species suitable for hydrogenation are selected from one or more transition metals from Groups 8, 9 or 10 of the Periodic Table, and compounds thereof; wherein the weight ratio of the one or more catalytic species comprising silver and tungsten therein to the one or more catalytic species suitable for hydrogenation is in the range of from 0.02:1 to 3000:1, on the basis of the total weight of the catalyst system.

In the catalyst system of the present invention, the one or more catalytic species comprising silver and tungsten therein may be conveniently selected from silver tungstate (Ag₂WO₄)-containing species and/or silver phosphotungstate (Ag₃PW₁₂O₄₀.xH₂O) -containing species.

Preferably, the one or more catalytic species comprising silver and tungsten therein are silver tungstate (Ag₂WO₄)-containing species.

In the catalyst system of the present invention, it is required that the one or more catalytic species which are suitable for hydrogenation have catalytic hydrogenation capabilities and are capable of catalysing the hydrogenation of material present in the reactor. The catalytic species which are suitable for hydrogenation may be present in elemental form or as one or more compounds. It is also suitable that these one or more catalytic species may be present in chemical combination with one or more other ingredients in the catalyst system.

In the catalyst system of the present invention, the one or more catalytic species which are suitable for the hydrogenation are selected from one or more transition metals from Groups 8, 9 or 10 of the Periodic Table, and compounds thereof. Preferably, said catalytic species may be one or more transition metals selected from the group of cobalt, iron, platinum, palladium, ruthenium, rhodium, nickel, iridium, and compounds thereof.

In one embodiment of the present invention, the one or more catalytic species suitable for hydrogenation are solid, unsupported species. Examples of such species include Raney Ni.

In another embodiment of the present invention, the one or more catalytic species suitable for hydrogenation are in homogeneous form.

In yet another embodiment of the present invention, the one or more catalytic species suitable for hydrogenation are on one or more solid catalyst supports.

The solid supports may be in the form of a powder or in the form of regular or irregular shapes such as spheres, extrudates, pills, pellets, tablets, monolithic structures. Alternatively, the solid supports may be present as surface coatings, for example, on the surfaces of tubes or heat exchangers.

Suitable solid support materials are those known to the skilled person and include, but are not limited to aluminas, silicas, zirconium oxide, magnesium oxide, zinc oxide, titanium oxide, carbon, activated carbon, zeolites, clays, silica alumina and mixtures thereof.

The one or more catalytic species comprising silver and tungsten therein may be present in the catalyst system in unsupported form or, alternatively, may also be present on an inert support. Examples of suitable supports include, but are not limited to, aluminas, silicas, zirconium oxide, magnesium oxide, zinc oxide, titanium oxide, carbon, activated carbon, zeolites, clays, silica alumina and mixtures thereof.

The weight ratio of the one or more species comprising silver and tungsten therein to the one or more catalytic species suitable for hydrogenation is in the range of from 0.02:1 to 3000:1, preferably in the range of from 0.1:1 to 100:1, on the basis of the total weight of the catalyst system.

The starting material for use in the process of the present invention comprises one or more saccharides selected from the group consisting of monosaccharides, disaccharides, oligosaccharides and polysaccharides. Examples of polysaccharides include cellulose, hemicelluloses, starch, glycogen, chitin and mixtures thereof. If the starting material comprises oligosaccharides or polysaccharides then, optionally, said starting material may be subjected to a pre-treatment before being fed to the reactor in a form that can be more conveniently converted in the process of the present invention. Suitable pre-treatment methods are known in the art and one or more may be selected from the group including, but not limited to, sizing, drying, grinding, hot water treatment, steam treatment, hydrolysis, pyrolysis, thermal treatment, chemical treatment, biological treatment.

Preferably, the starting material for use in the process of the present invention comprises one or more saccharides selected from the group consisting of glucose, sucrose and starch. Said saccharides are suitably present as a solution, a suspension or a slurry in solvent.

The solvent present in the reactor is not limited and may be conveniently selected from water, C1 to C6 alcohols, ethers, and other suitable organic compounds, and mixtures thereof. Preferably, the solvent is water. If the starting material is provided to the reactor as a solution, suspension or slurry in a solvent, said solvent is also suitably water or a C1 to C6 alcohol, ether, and other suitable organic compounds, and mixtures thereof. Preferably, both solvents are the same. More preferably, both solvents comprise water. Most preferably, both solvents are water.

In the process of the present invention, the one or more catalytic species comprising silver and tungsten therein are typically employed in the catalyst system in an amount in the range of from 0.001 to 10 wt. % more preferably in an amount in the range of from 0.001 to 6 wt. %, and most preferably in an amount in the range of from 0.001 to 3 wt. %, based on the total weight of the reaction mixture.

By "reaction mixture" in the present invention, is meant the total weight of the starting material, catalyst system and solvent present in the reactor.

The temperature in the reactor is suitably at least 130 °C, preferably at least 150°C, more preferably at least 170 °C, even more preferably greater than 190 °C and most preferably at least 195 °C. The temperature in the reactor is suitably at most 300 °C, preferably at most 280 °C, more preferably at most 270 °C, even more preferably at most 250 °C. It is particularly preferred that the reactor temperature is in the range of from 130 to 300 °C, more preferably in the range of from 150 to 270 °C, and most preferably in the range of from 195 to 250 °C. Preferably, the reactor is heated to a temperature within these limits before addition of any starting material and is maintained at such a temperature until all reaction is complete.

The pressure in the reactor is generally at least 1 MPa, preferably at least 2 MPa, more preferably at least 3 MPa. The pressure in the reactor is generally at most 25 MPa, more preferably at most 20 MPa, more preferably at most 18 MPa. Preferably, the reactor is pressurised to a pressure within these limits by addition of hydrogen before addition of any starting material and is maintained at such a pressure until all reaction is complete. This can be achieved by subsequent addition of hydrogen.

The process of the present invention takes place in the presence of hydrogen. Preferably, the process of the present reaction takes place in the absence of air or oxygen. In order to achieve this, it is preferable that the atmosphere in the reactor be evacuated and replaced with hydrogen repeatedly, after loading of any initial reactor contents. It may also be suitable to add further hydrogen to the reactor as the reaction proceeds.

The reactor in the present invention may be any suitable reactor known in the art.

The process may be carried out as a batch process or as a continuous flow process.

In one embodiment of the invention, the process is a batch process. In such a process, the reactor may be loaded with the catalyst system, solvent and one or more saccharides, and the reactor may then be pressurised with hydrogen at room temperature, sealed and heated to the reaction temperature.

In semi-continuous processes, the same process is carried out. After holding the reactor at temperature for a given duration, the reactor is then cooled down, opened, sampled for analysis and subsequently another portion of the starting material is added and retested. This may be repeated multiple times, typically until the total concentration of the saccharide in the solvent is at least 5 wt. %.

"Total concentration" as used herein refers to the concentration calculated as a weight percentage of the total amount of saccharide added in the total amount of solvent present in the reactor. The total amount of saccharide added corresponds to the sum total of the amount of saccharide added in the first portion and all further portions, if any. The total amount of solvent in the reactor includes any solvent already present in the reactor as well as any solvent present in the slurry, solution or suspension of the starting material. Preferably, further portions of starting material are added to the reactor over time until the total concentration of one or more saccharides in the solvent in the reactor is at least 5 wt. %, more preferably at least 8 wt. %, even more preferably at least 10 wt. %. Suitably, the total concentration of the one or more saccharides in the solvent is no higher than 50 wt. %, preferably no higher than 40 wt. %.

In embodiments of the invention, addition of further portions of starting material may occur in a continuous manner or the portions may be added in a discontinuous manner with time elapsing between the end of the addition of one portion and the start of the addition of the next portion. In the embodiments of the invention wherein the portions are added in a discontinuous manner, the number and size of each portion will be dependent on the scale of the reactor. Preferably, the total number of portions including the first portion is no less than 5, more preferably no less than 8, even more preferably no less than 10. The amount of time over which each portion is added and the time to be elapsed between the end of the addition of one portion and the start of the addition of the next portion will also depend on the scale of the reactor. Preferably, the time to be elapsed between the end of the addition of one portion and the start of the addition of the next portion will be greater than the amount of time over which each portion is added.

In embodiments of the invention wherein the process is a batch process, after addition of all of the portions of the starting material, the reaction may then be allowed to proceed to completion for a further period of time. The reaction product will then be removed from the reactor or the reactor product can be sampled in between the additions of the starting material.

In embodiments of the invention wherein the process is carried out as a continuous flow process, after initial loading of some or all of the catalysts and, optionally, solvent, the reactor is heated and pressurised with hydrogen and then the first portion of starting material is introduced into the reactor. Further portions of starting material are then provided to the reactor. Reaction product is removed from the reactor in a continuous manner. In some embodiments of the invention, catalysts may be added in a continuous manner.

In embodiments of the present invention, the starting material is suitably a saccharide feedstock comprising at least 1 wt. % saccharide as a solution, suspension or slurry in a solvent. Preferably, said saccharide feedstock comprises at least 2 wt. %, more preferably at least 5 wt. %, even more preferably at least 10 wt. %, most preferably at least 20 wt. % saccharide in a solvent. Suitably, the saccharide feedstock contains no more than 50 wt. %, preferably no more than 40 wt. % saccharide in a solvent.

The weight ratio of catalyst system to saccharides in the starting material is suitably in the range of from 1:100 to 1:10000.

Figure 1 is a schematic diagram of an exemplary, but non-limiting, embodiment of the process of the invention. A feed 101 comprising polysaccharides and solvent is provided to a pre-treatment unit 102 to convert it mainly into glucose, sucrose and/or starch in solvent to form feed 103. The pre-treatment unit 102 may consist of multiple pre-treatment units performing the same or different pre-treatment functions. Pre-treatment is an optional step in case the feed is polysaccharide. Feed 103 is then fed to the main reactor 104 where it undergoes hydrogenation/hydrogenolysis in the presence of the catalyst system to produce a product stream 105 comprising MEG.

The process of the present invention is not limited to any particular reactor or flow configurations, and those depicted in Figure 1 are merely exemplary. Furthermore, the sequence in which various feed components are introduced into the process and their respective points of introduction, as well as the flow connections, may be varied from that depicted in Figure 1.

The invention is further illustrated by the following Examples.

### Examples

75 ml Hastelloy C batch autoclaves, with magnetic stir bars, were used to screen various conditions, catalysts and feedstocks.

In typical experiments, known weights of catalysts and feedstocks were added to the autoclaves along with 30 ml of the solvent (typically water).

If the catalysts or feedstocks were present as slurries or solutions, the total volume of those as well as the solvent was kept at 30 ml.

### Example 1

### Methodology

In Example 1, 0.3 g of glucose was dissolved in 30 ml of water. Catalysts were also added to the solution. The loaded autoclave was then purged three times with nitrogen, followed by hydrogen purge.

The hydrogen pressure was then raised to 2000 psig or ∼14 MPa of hydrogen and the autoclave was sealed and left stirring overnight to do a leak test.

The next morning the autoclave was de-pressurised to the target hydrogen pressure (1450 psig or 10.1 MPa) at room temperature, and closed. Next, the temperature was ramped to the target run temperature (195 °C) either as a fast ramp or in steps.

In Example 1, there was a fast ramp to temperature. The autoclave was held at the target temperature for known durations of time (75 min), while both the temperature and pressure were monitored. After the required run time had elapsed, the heating was stopped, and the reactor was cooled down to room temperature, de-pressurised, purged with nitrogen and then opened.

The contents of the autoclave were then analyzed via Gas Chromatography (GC) or High Pressure Liquid Chromatography (HPLC) after being filtered. The yields of the various components were measured as wt. % basis the total saccharide loaded.

Table 1 provides details on the catalyst systems tested in Example 1 using 1 wt. % glucose as the feedstock in 30 ml of water. Catalyst systems A to D are comparative in nature and are known in the art as suitable tungsten-containing catalyst systems for the conversion of saccharide-containing feedstocks to monoethylene glycol. Catalyst systems E and F are according to the present invention.

**Table 1**

| Catalyst System | Hydrogenolysis Catalyst (a) | | Hydrogenation Catalyst (b) | | Ratio (a) : (b) |
|---|---|---|---|---|---|
| | Component | Amount g | Component | Amount g | |
| A (Comp) | Tungstic acid (H₂WO₄) | 0.0672 | 1 % Ru on SiO₂ | 0.045 | 1.5 |
| B (Comp) | 10 % W, 2 % Mo on Zr02 | 0.025 | 1 % Ru on SiO₂ | 0.025 | 1.0 |
| C (Comp) | 10 % W on TiO₂ | 0.025 | 1 % Ru on SiO₂ | 0.025 | 1.0 |
| D (Comp) | 10 % W on TiO₂ | 0.025 | Raney Ni 2800 | 0.008 | 3.1 |
| E | Silver tungstate | 0.0417 | 1 % Ru on SiO₂ | 0.125 | 0.3 |
| F | Silver tungstate | 0.0417 | Raney Ni 2800 | 0.017 | 2.5 |

### Discussion

It is apparent from Table 2 that Catalyst Systems E and F, according to the present invention not only give the highest yields of monoethylene glycol but also the largest total yield of monoethylene glycol, monopropylene glycol and hydroxyacetone.

Surprisingly, use of Catalyst System F results in a very high C2:C3 ratio (MEG:(MPG+HA)).

**Table 2**

| Catalyst System | MEG | MPG | * HA | ** 1,2-BDO | *** 1H2BO | MEG: (MPG+HA) |
|---|---|---|---|---|---|---|
| | wt. % | wt. % | wt. % | wt. % | wt. % | |
| A (Comp) | 19.3 | 2.6 | 10.2 | 2.8 | 14.7 | 1.5 |
| B (Comp) | 12.0 | 0.0 | 9.9 | 2.2 | 13.8 | 1.2 |
| C (Comp) | 33.9 | 4.8 | 2.9 | 1.9 | 2.7 | 4.5 |
| D (Comp) | 15.5 | 2.1 | 0.6 | 1.3 | 0.7 | 5.8 |
| E | 41.0 | 8.9 | 1.4 | 1.8 | 2.9 | 4.0 |
| F | 41.3 | 2.5 | 1.5 | 1.4 | 2.4 | 10.3 |

| | | | | | | |
|---|---|---|---|---|---|---|
| * hydroxyacetone ** 1,2-butanediol *** 1-hydroxy-2-butanone | | | | | | |

### Example 2

### Methodology

The same methodology as described in Example 1 was used, except that after the reactor was cooled, a 1 ml product sample was taken for analysis. Subsequently 1 wt. % of the starting saccharide material along with 1 ml of water was added to the reactor, sealed and subjected to the same testing methodology as described in Example 1.

This procedure was repeated 6 times so that the total saccharide concentration was 6 wt. %.

The yields are reported relative to the total amount of saccharide up to that addition.

Table 3 provides details on the catalyst systems tested in Example 2.

**Table 3**

| Catalyst System | Hydrogenolysis Catalyst (a) | | | Hydrogenation Catalyst (b) | | Ratio (a) : (b) |
|---|---|---|---|---|---|---|
| | Component | Amount (g) | Amount of W (g) | Component | Amount (g) | |
| G | Silver tungstate | 0.0417 | 0.017 | Raney Ni 2800 | 0.023 | 1.8 |
| H(Comp) | Sodium phosphotungstate | 0.023 | 0.017 | Raney Ni 2800 | 0.021 | 1.1 |

Table 3 provides details on the catalyst systems tested in Example 2 using 1 wt. % corn starch as the feedstock in 30 ml of water, pressurized to 1450 psig or 10 MPa at room temperature with hydrogen, then ramped to 195 °C and held at that temperature for 75 min.

The corn starch used in Example 2 had 231 ppm sulphur, 173 ppm phosphorus and 19 ppm of chlorine contaminants present as different forms of those elements.

Catalyst system H is comparative in nature. Catalyst system G is according to the present invention. Both catalyst systems have equivalent amounts of tungsten and Raney Ni 2800 loading.

### Results

Table 4 presents the results of Example 2.

**Table 4**

| Catalyst System | Addition of 1 wt. % Corn Starch | MEG (wt. %) | MPG (wt. %) | HA* (wt. %) | 1,2-BDO** (wt. %) | 1H2BO*** (wt. %) | MEG: (MPG+HA) | Catalyst Loss (%) | MEG Yield Loss (%) |
|---|---|---|---|---|---|---|---|---|---|
| G | 1 | 53.2 | 5.7 | 4.3 | 4.5 | 6.8 | 5.3 | | |
| | 2 | 49.1 | 4.4 | 3.4 | 4 .4 | 5.8 | 6.3 | 3.3 | -7.8 |
| | 3 | 45.8 | 3.8 | 3.0 | 4.4 | 5.4 | 6.7 | 3.3 | -6.6 |
| | 4 | 38.4 | 3.4 | 2.9 | 4.4 | 4.7 | 6.1 | 3.3 | -16.3 |
| | 5 | 31.7 | 2.9 | 2.6 | 3.9 | 3.6 | 5.7 | 6.7 | -17.3 |
| | 6 | 25.5 | 2.4 | 1.9 | 3.1 | 2.0 | 5.9 | 3.3 | -19.6 |
| H (comp) | 1 | 21.2 | 2.0 | 1.5 | 2.6 | 1.2 | 6.0 | | |
| | 2 | 43.0 | 5.9 | 3.2 | 4.3 | 4.3 | 4.8 | 3.3 | |
| | 3 | 31.2 | 3.6 | 4.0 | 3.1 | 4.1 | 4 .1 | 3.3 | -27.5 |
| | 4 | 21.4 | 2.5 | 3.1 | 2.3 | 2.5 | 3.8 | 3.3 | -31.3 |
| | 5 | 15.9 | 2.0 | 2.3 | 1.7 | 1.4 | 3.7 | 3.3 | -25.8 |
| | 6 | 11.9 | 1.5 | 1.7 | 1.3 | 0.9 | 3.7 | 3.3 | -25.2 |

| | | | | | | | | | |
|---|---|---|---|---|---|---|---|---|---|
| * hydroxyacetone ** 1,2-butanediol *** 1-hydroxy-2-butanone | | | | | | | | | |

### Discussion

It is apparent from Table 4 that Catalyst System G, according to the present invention is more resistant to contaminants than the comparative Catalyst System H. Both catalyst systems have equivalent amounts of tungsten and Raney Ni 2800 catalysts. However, Catalyst System G containing silver tungstate resulted in less MEG yield decline over 6 additions of 1 wt. % corn starch than Catalyst System H containing sodium phosphotungstate.

That is to say, Catalyst System G according to the present invention clearly shows superior tolerance to various feed stock contaminants. Furthermore, Catalyst System G not only has higher yields of MEG as well as MEG+MPG+HA, but also results in a higher ratio of MEG:(MPG + HA).

### Example 3

### Methodology

The same methodology as described in Example 1 was used to test a catalyst system comprising supported silver tungstate.

0.166 g of 10 wt.% silver tungstate on titania (TiO₂) and 0.125 g of 1 wt. % Ru on SiO₂ was used with 1 wt. % glucose as the feedstock in 30 ml of water, pressurized to 1450 psig or 10 MPa at room temperature with hydrogen, then ramped to 195 °C and held at that temperature for 75 min. Catalyst systems E and I are according to the present invention and are detailed in Table 5.

**Table 5**

| Catalyst System | Hydrogenolysis Catalyst (a) | | Hydrogenation Catalyst (b) | | Ratio (a) : (b) |
|---|---|---|---|---|---|
| | Component | Amount (g) | Component | Amount (g) | |
| E | Silver tungstate | 0.0417 | 1 % Ru on SiO₂ | 0.125 | 0.3 |
| I | 10 % Silver tungstate on Ti02 | 0.166 | 1 % Ru on SiO₂ | 0.125 | 1.3 |

### Results

Table 6 presents the results of Example 3.

**Table 6**

| Catalyst System | MEG | MPG | HA* | 1,2-BDO** | 1H2BO *** | MEG: (MPG+HA) |
|---|---|---|---|---|---|---|
| | (wt. %) | (wt. %) | (wt. %) | (wt. %) | (wt. %) | |
| E | 41.0 | 8.9 | 1.4 | 1.8 | 2.9 | 4.0 |
| I | 39.7 | 4.0 | 0.0 | 2.5 | 1.1 | 9.9 |

| | | | | | | |
|---|---|---|---|---|---|---|
| * hydroxyacetone ** 1,2-butanediol *** 1-hydroxy-2-butanone | | | | | | |

### Discussion

It is apparent from Table 6 that the hydrogenolysis catalyst supplied to the reactor as supported and unsupported silver tungstate are both effective catalysts in the catalyst system of the present invention. The resulting catalyst systems both give high yields of MEG.

## Claims

1. A catalyst system comprising:
a) one or more catalytic species comprising silver and tungsten therein,
wherein the one or more catalytic species comprising silver and tungsten therein are selected from silver tungstate-containing species and/or silver phosphotungstate -containing species; and
b) one or more catalytic species suitable for hydrogenation, wherein the one or more catalytic species suitable for hydrogenation are selected from one or more transition metals from Groups 8, 9 or 10 of the Periodic Table, and compounds thereof;
wherein the weight ratio of the one or more catalytic species comprising silver and tungsten therein to the one or more catalytic species suitable for hydrogenation is in the range of from 0.02:1 to 3000:1, on the basis of the total weight of the catalyst system.

2. Catalyst system according to Claim 1, wherein the one or more catalytic species comprising silver and tungsten therein are selected from silver tungstate (Ag₂WO₄)-containing species and/or silver phosphotungstate (Ag₃PW₁₂O₄₀.xH₂O) -containing species.

3. Catalyst system according to any one of Claims 1 to 2, wherein the one or more catalytic species suitable for hydrogenation are selected from one or more transition metals selected from the group of cobalt, iron, platinum, palladium, ruthenium, rhodium, nickel, iridium, and compounds thereof.

4. Catalyst system according to any one of Claims 1 to 3, wherein the one or more catalytic species suitable for hydrogenation are solid, unsupported species.

5. Catalyst system according to any one of Claims 1 to 4, wherein the one or more catalytic species comprising silver and tungsten therein and/or the one or more catalytic species suitable for hydrogenation are on a solid catalyst supports.

6. Catalyst system according to Claim 5, wherein the solid catalyst supports are selected from aluminas, silicas, zirconium oxide, magnesium oxide, zinc oxide, titanium oxide, carbon, activated carbon, zeolites, clays, silica alumina and mixtures thereof.

7. Catalyst system according to any one of Claims 1 to 6, wherein the weight ratio of the one or more catalytic species comprising silver and tungsten therein to the one or more catalytic species suitable for hydrogenation is in the range of from 0.1:1 to 100:1, on the basis of the total weight of the catalyst system.

8. A process for the preparation of monoethylene glycol from starting material comprising one or more saccharides, by contacting said starting material with hydrogen in a reactor in the presence of a solvent and a catalyst system according to any one of Claims 1 to 7.

9. Process according to Claim 8, wherein the saccharides are selected from the group consisting of monosaccharides, disaccharides, oligosaccharides and polysaccharides.

10. Process according to Claim 8 or 9, wherein the one or more catalytic species comprising silver and tungsten therein are present in an amount in the range of from 0.001 to 10 wt. % based on the total weight of the reaction mixture.

11. Process according to any one of Claims 8 to 10, wherein the reactor temperature is in the range of from 130 to 300 °C.

12. Process according to any one of Claims 8 to 11, wherein the reactor pressure is in the range of from at least 1 to at most 25 MPa.

## Patentansprüche

1. Katalysatorsystem, das Folgendes umfasst:
a) eine oder mehrere katalytische Spezies, die Silber und Wolfram darin umfassen,
wobei die eine oder die mehreren katalytischen Spezies, die Silber und Wolfram darin umfassen, aus Silberwolframat enthaltenden Spezies und/oder Silberphosphowolframat enthaltenden Spezies ausgewählt sind; und
b) eine oder mehrere katalytische Spezies, die für eine Hydrierung geeignet sind,
wobei die eine oder die mehreren katalytischen Spezies, die für die Hydrierung geeignet sind, aus einem oder mehreren Übergangsmetallen aus den Gruppen 8, 9 oder 10 des Periodensystems und Verbindungen davon ausgewählt sind;
wobei das Gewichtsverhältnis der einen oder der mehreren katalytischen Spezies, die Silber und Wolfram darin umfassen, zu der einen oder den mehreren katalytischen Spezies, die für die Hydrierung geeignet sind, in dem Bereich von 0,02 : 1 bis 3000 : 1 liegt, auf der Basis des Gesamtgewichts des Katalysatorsystems.

2. Katalysatorsystem nach Anspruch 1, wobei die eine oder die mehreren katalytischen Spezies, die Silber und Wolfram darin umfassen, aus Silberwolframat (Ag₂WO₄) enthaltenden Spezies und/oder Silberphosphowolframat (Ag₃^{PW}12°40.xH₂O) enthaltenden Spezies ausgewählt sind.

3. Katalysatorsystem nach einem der Ansprüche 1 bis 2, wobei die eine oder die mehreren katalytischen Spezies, die für die Hydrierung geeignet sind, aus einem oder mehreren Übergangsmetallen ausgewählt sind, die aus der Gruppe von Cobalt, Eisen, Platin, Palladium, Ruthenium, Rhodium, Nickel, Iridium und Verbindungen davon ausgewählt sind.

4. Katalysatorsystem nach einem der Ansprüche 1 bis 3, wobei die eine oder die mehreren katalytischen Spezies, die für die Hydrierung geeignet sind, feste, nicht getragene Spezies sind.

5. Katalysatorsystem nach einem der Ansprüche 1 bis 4, wobei die eine oder die mehreren katalytischen Spezies, die Silber und Wolfram darin umfassen, und/oder die eine oder die mehreren katalytischen Spezies, die für die Hydrierung geeignet sind, sich auf einem festen Katalysatorträgern befinden.

6. Katalysatorsystem nach Anspruch 5, wobei die festen Katalysatorträger aus Aluminiumoxiden, Siliciumdioxiden, Zirconiumoxid, Magnesiumoxid, Zinkoxid, Titanoxid, Kohlenstoff, aktivem Kohlenstoff, Zeolithen, Tonen, Siliciumdioxidaluminiumoxid und Gemischen davon ausgewählt sind.

7. Katalysatorsystem nach einem der Ansprüche 1 bis 6, wobei das Gewichtsverhältnis der einen oder der mehreren katalytischen Spezies, die Silber und Wolfram darin umfassen, zu der einen oder den mehreren katalytischen Spezies, die für die Hydrierung geeignet sind, in dem Bereich von 0,1 : 1 bis 100 : 1 liegt, auf der Basis des Gesamtgewichts des Katalysatorsystems.

8. Vorgang für die Herstellung von Monoethylenglycol aus einem Ausgangsmaterial, das ein oder mehrere Saccharide umfasst, durch Inberührungbringen des Ausgangsmaterials mit Wasserstoff in einem Reaktor in der Gegenwart eines Lösungsmittels und eines Katalysatorsystems nach einem der Ansprüche 1 bis 7.

9. Vorgang nach Anspruch 8, wobei die Saccharide aus der Gruppe ausgewählt sind, die aus Monosacchariden, Disacchariden, Oligosacchariden und Polysacchariden besteht.

10. Vorgang nach Anspruch 8 oder 9, wobei die eine oder die mehreren katalytischen Spezies, die Silber und Wolfram darin umfassen, in einer Menge in dem Bereich von 0,001 bis 10 Gew.-% basierend auf dem Gesamtgewicht des Reaktionsgemischs vorhanden sind.

11. Vorgang nach einem der Ansprüche 8 bis 10, wobei die Reaktortemperatur in dem Bereich von 130 bis 300 °C liegt.

12. Vorgang nach einem der Ansprüche 8 bis 11, wobei der Reaktordruck in dem Bereich von wenigstens 1 bis höchstens 25 MPa liegt.

## Revendications

1. Système catalytique comprenant :
a) une ou plusieurs espèces catalytiques comprenant de l'argent et du tungstène en leur sein,
la ou les espèces catalytiques comprenant de l'argent et du tungstène en leur sein étant choisies parmi les espèces contenant du tungstate d'argent et/ou les espèces contenant du phosphotungstate d'argent ; et
b) une ou plusieurs espèces catalytiques appropriées pour l'hydrogénation,
la ou les espèces catalytiques appropriées pour l'hydrogénation étant des espèces solides non supportées choisies parmi un ou plusieurs métaux de transition des groupes 8, 9 ou 10 du tableau périodique, et des composés de ceux-ci ;
le rapport en poids de la ou des espèces contenant de l'argent et du tungstène en leur sein à la ou aux espèces catalytiques appropriées pour l'hydrogénation étant compris entre 0,02:1 et 3000:1, sur la base du poids total du système catalytique.

2. Système catalytique selon la revendication 1, la ou les espèces catalytiques comprenant de l'argent et du tungstène en leur sein étant choisies parmi les espèces contenant du tungstate d'argent (Ag₂WO₄) et/ou les espèces contenant du phosphotungstate d'argent (Ag₃PW₁₂O₄₀.xH₂O).

3. Système catalytique selon l'une quelconque des revendications 1 ou 2, la ou les espèces catalytiques appropriées pour l'hydrogénation étant choisies parmi un ou plusieurs métaux de transition choisis dans le groupe constitué par le cobalt, le fer, le platine, le palladium, le ruthénium, le rhodium, le nickel, l'iridium et des composés de ceux-ci.

4. Système catalytique selon l'une quelconque des revendications 1 à 3, la ou les espèces catalytiques appropriées pour l'hydrogénation étant des espèces solides non supportées.

5. Système catalytique selon l'une quelconque des revendications 1 à 4, la ou les espèces catalytiques comprenant de l'argent et du tungstène en leur sein et/ou la ou les espèces catalytiques appropriées pour l'hydrogénation étant sur un supports de catalyseur solide.

6. Système catalytique selon la revendication 5, les supports catalytiques solides étant choisis parmi les alumines, les silices, l'oxyde de zirconium, l'oxyde de magnésium, l'oxyde de zinc, l'oxyde de titane, le carbone, le charbon actif, les zéolites, les argiles, la silice-alumine et des mélanges de ceux-ci.

7. Système catalytique selon l'une quelconque des revendications 1 à 6, le rapport en poids de la ou des espèces contenant du phosphotungstate de sodium en leur sein à la ou aux espèces catalytiques appropriées pour l'hydrogénation étant compris entre 0,1:1 et 100:1, sur la base du poids total du système catalytique.

8. Procédé de préparation de monoéthylène glycol à partir d'un produit de départ comprenant un ou plusieurs saccharides, par mise en contact dudit produit de départ avec de l'hydrogène dans un réacteur en présence d'un solvant et d'un système catalytique selon l'une quelconque des revendications 1 à 7.

9. Procédé selon la revendication 8, les saccharides étant choisis dans le groupe constitué par les monosaccharides, disaccharides, oligosaccharides et polysaccharides.

10. Procédé selon la revendication 8 ou 9, la ou les espèces comprenant de l'argent et du tungstène en leur sein étant présentes en une quantité comprise dans la plage de 0,001 à 10 % en poids, sur la base du poids total du mélange réactionnel.

11. Procédé selon l'une quelconque des revendications 8 à 10, la température du réacteur étant comprise dans la plage de 130 à 300 °C.

12. Procédé selon l'une quelconque des revendications 8 à 11, la pression du réacteur étant comprise dans la plage d'au moins 1 à au plus 25 MPa.
